(19) **Europäisches Patentamt**

**European Patent Office**

**Office européen des brevets**

(11) Publication number: **0 307 520 B1**

# EUROPEAN PATENT SPECIFICATION

(45) Date of publication of patent specification: **12.06.91**

(51) Int. Cl.⁵: **B01J 23/74**, B01J 35/10, B01J 27/04, C07C 5/05

(21) Application number: **87308086.5**

(22) Date of filing: **14.09.87**

(54) **High macropore selective hydrogenation catalyst.**

(43) Date of publication of application:
**22.03.89 Bulletin 89/12**

(45) Publication of the grant of the patent:
**12.06.91 Bulletin 91/24**

(84) Designated Contracting States:
**AT BE DE ES FR GB GR IT NL SE**

(56) References cited:
**FR-A- 1 523 687**
**US-A- 3 472 763**
**US-A- 3 793 388**
**US-A- 3 959 400**
**US-A- 4 695 560**

(73) Proprietor: **UOP (a New York general partner-ship)**
**25 East Algonquin Road**
**Des Plaines Illinois 60017-5017(US)**

(72) Inventor: **Gattuso, Mark J.**
**1029 West Austin Lane**
**Palatine Illinois 60067(US)**
Inventor: **Ellig, Daniel L.**
**1651 Thacker Street**
**Des Plaines 60016(US)**

(74) Representative: **Brock, Peter William**
**URQUHART-DYKES & LORD 91 Wimpole Street**
**London W1M 8AH(GB)**

## Description

This invention generally relates to a catalyst composition for use in a hydrocarbon conversion process. The invention is directly concerned with a composition comprising a solid catalyst useful in converting diolefinic hydrocarbons into monoolefinic hydrocarbons. Such compositions find utility in treating the butene feed streams to motor fuel alkylation process units and in decreasing the concentration of diolefinic hydrocarbons in the product stream from a dehydrogenation reaction. This invention is specifically directed to a selective hydrogenation catalyst which comprises an alumina base or support material having a unique pore structure, with nickel and sulphur deposited upon the support material.

It has been recognized in the petrochemical and refining industries that in some instances, it is desirable selectively to convert diolefinic hydrocarbons into monoolefinic hydrocarbons. The economic advantage of this conversion has prompted the development of a significant number of catalysts for this purpose. Many of these catalysts employ a traditional hydrogenation catalyst metal, such as nickel, platinum, or palladium, on a solid support material such as alumina. For instance, US-A-3234298 teaches the use of a sulphided nickel on alumina catalyst for a selective hydrogenation of diene-containing cracked hydrocarbon oils. US-A-3472763 is pertinent for its teaching of a selective hydrogenation catalyst which comprises from 1 to 20 percent of nickel (as nickel oxide) on alumina. This reference indicates that it is preferable to pretreat the catalyst with a sulphur compound, and specifies preferred pore size distributions.

US-A-3662015 is pertinent for its teaching of a nickel on alumina selective hydrogenation catalyst. US-A-4440956 is directed to a catalyst for use in removing acetylenes from liquid hydrocarbon streams containing diolefinic hydrocarbons without substantially decreasing the percentage of diolefinic hydrocarbons in the feed material. This reference is believed pertinent for its teaching of the various aluminas which may be used in conjunction with the common hydrogenation metals in producing such a selective catalyst. US-A-3919341 is pertinent for its teaching of a sulphided nickel on alumina composite which is basically intended to function as an olefin-isomerization catalyst. The test results reported in Table 2 and column 12 indicate a selective hydrogenation of butadiene, however. US-A-4469907 is believed pertinent for its general teaching of an improved method of selective hydrogenation, and US-A-3696160 is believed pertinent for its teaching of the desirability of using selective hydrogenation upstream of, or in conjunction with, an alkylation process in which $C_3$ and $C_4$ olefinic hydrocarbons are consumed for the production of motor fuel.

US-A-4179408 describes the preparation of spherical alumina catalyst supports having a large portion of the total pore volume present in macropores greater than 1000 Angstroms (columns 20-22). US-A-4179411 describes an alumina of specific pore size distribution.

This invention is concerned with the problem of providing a catalyst suitable for the selective hydrogenation of diolefinic hydrocarbons to monoolefinic hydrocarbons. The catalyst according to the invention providing the solution to this problem exhibits outstanding selectivity in the conversion of diolefins having more than 8 carbon atoms, thereby increasing the yield of monoolefinic hydrocarbons in the product stream. The catalyst comprises an alumina support material, from 1.0 to 25 wt. %, preferably 4.1 to 25 wt. %, of nickel and 0.05 to 1.5 wt. % of sulphur. Preferably it is substantially free of halogens and noble metals. The catalyst is characterized by a lack of micropores and an abundance of macropores. Less than 25 percent of the total pore volume of the catalyst is provided by pores having pore diameters less than 150 Angstroms and more than 60 percent is provided by macropores having pore diameters greater than 600 Angstroms. The invention also provides a selective hydrogenation process employing the above-defined catalyst.

The advantages provided by the catalyst according to this invention are illustrated by the accompanying Drawings, in which:

Figure 1 is a graph presenting data on the volume percentage of diolefins in the effluent stream of a pilot plant testing the catalyst of the invention and two reference catalysts.

Figure 2 is a similar graph which gives the concentration of monoolefins in the effluent stream during the same tests.

Many petrochemical processes either produce or consume monoolefinic hydrocarbons. In many of these processes, diolefinic hydrocarbons are considered as impurities. As an or in a substantially pure olefinic stream. The catalyst of the present invention may also be used in a purification step performed on the effluent of an olefin separation process, such as the separation of olefinic hydrocarbons from a mixture of olefinic and paraffinic hydrocarbons through the use of selective adsorption techniques.

Accordingly, the catalyst of the instant invention is designed for the selective hydrogenation of diolefinic hydrocarbons to monoolefinic hydrocarbons and comprises an example, in the production of linear alkylbenzene in an integrated process, such as that of US-A-34844981, monoolefinic hydrocarbons are produced in a dehydrogenation zone and then passed into an alkylation zone. The olefins are therein reacted with benzene to produce the linear alkylaromatic hydrocarbon product. There is some unavoidable production of diolefinic hydrocarbons in the dehydrogenation zone, and these diolefinic hydrocarbons would normally be present in the olefins charged to the alkylation zone. The presence of the diolefinic straight chain material results in the production of undesirable by-products, such as various biphenyl compounds. These are impurities which impart undesirable properties to the intended linear alkylbenzene product. The presence of diolefinic hydrocarbons results in the production of undesirable high boiling point by-products, and an increased rate of acid consumption. The selective hydrogenation catalyst of this invention can be employed in a process to remedy these situations and others in which diolefinic hydrocarbons are considered an undesired contaminant in a mixture of monoolefins and paraffins, alumina support material, from 0.05 to 1.5 wt. % of sulphur, and 1.0 to 25 wt. % of nickel. The alumina support material has a total pore volume from 1.4 to 3 cm³/g, and a surface area greater than 150 m²/g, with less than 25 percent of the total pore volume being provided by pores having pore diameters of less than 150 Angstroms and with over 60 percent of the pore volume being provided by pores having pore diameters greater than 600 Angstroms.

The feed stream processed by the above-defined catalyst may comprise a mixture of different hydrocarbons having the same number of carbons atoms per molecule, or a mixture of hydrocarbons having a significant range in carbon numbers. For instance, the feed stream processed with this catalyst may contain substantially only $C_4$ or $C_5$ hydrocarbons. Alternatively, the feed stream may comprise a mixture of $C_8$ to $C_{15}$ hydrocarbons including paraffinic hydrocarbons, monoolefinic hydrocarbons, and diolefinic hydrocarbons. In general, it is believed the catalyst of this invention should be most effective in treating hydrocarbons having from 8 to 20 carbon atoms per molecule.

Selective hydrogenation processes are normally performed under relatively mild hydrogenation conditions. These conditions will normally result in the hydrocarbons being present in the liquid phase, the reactants normally being maintained under the minimum pressure sufficient to keep the reactants in the liquid phase. A broad range of suitable operating pressures, therefore, is from 40 to 800 psig (276 to 5516 k Pa g), with a pressure between 50 and 300 psig (345 to 2069 k Pa g) being preferred. A relatively moderate temperature from 25 to 350°C (77 to 662°F) should be employed. Preferably, the temperature of the hydrogenation zone is maintained at from 50 to 200°C (122 to 392°F). The liquid hourly space velocity of the reactants through the catalyst should be above 1 hr⁻¹. Preferably, it is above 5, and more preferably it is between 5 and 35 hr⁻¹. Another variable operating condition is the ratio of hydrogen to diolefinic hydrocarbons within the selective hydrogenation zone. The amount of hydrogen required to achieve a certain conversion is believed to depend upon both reactor temperature and the molecular weight of the feed hydrocarbons. To avoid the undesired saturation of a significant amount of monoolefinic hydrocarbons, there should be less than twice the stoichiometric amount of hydrogen required for the selective hydrogenation to monoolefinic hydrocarbons of the diolefinic hydrocarbons which are present in the liquid phase process stream. Preferably, the mole ratio of hydrogen to diolefinic hydrocarbons in the material entering the catalyst bed is between 1:1 and 1.8:1. In some instances, it may be desirable to operate with a less than stoichiometric amount of hydrogen, with mole ratios down to 0.75:1 being acceptable. The optimum set of conditions will of course vary, depending on such factors as the compositions of the feed stream and the degree of saturation of diolefinic hydrocarbons which it is desired to bring about.

The catalyst of this invention is preferably employed in a fixed bed reactor containing a cylindrical bed of catalyst, through which the reactants move in a vertical direction. It is preferred that the reactants flow upwards through the reactor, as this provides good mixing. The catalyst may be present within the reactor as pellets, spheres, extrudates, irregular shaped granules, etc. The reactants would be preferably brought up to the desired inlet temperature of the reaction zone, mixed with hydrogen and then passed into and through the reactor. Alternatively, the reactants may be mixed with the desired amount of hydrogen and then heated to the desired inlet temperature. In either instance, the effluent of the reaction zone may be passed into a product recovery facility for the removal of residual hydrogen, or may be passed directly into downstream product utilization zones if the presence of the residual hydrogen is acceptable. Hydrogen may be removed by flashing the effluent stream to a lower pressure or by passing the effluent stream into a stripping column.

The preferred form of the catalyst is spheres having a diameter from 1/64 to ¼-inch (0.4 to 6.4 mm). Spheres of solid catalyst support material can be made in a number of different ways including rolling and compaction techniques. It is greatly preferred, however, that the spherical alumina particles are formed by a method for effecting gelation of a alumina sol such as described in US-A-2620314. This method of gelation of alumina to form spheres is commonly known in the art as the oil drop method. The alumina sol may be also be formed a number of different ways. A typical way is to digest aluminium metal with an aqueous solution of approximately 12% hydrogen chloride to produce an aluminium chloride sol. Another method comprises electrolysis of a solution of aluminium chloride in an electrolytic cell. A common method of preparing an alumina sol is the addition of aluminium metal to an aqueous solution of aluminium chloride, with this mixture being subjected to heating and digesting at its boiling point. The method by which the alumina sol is prepared is not intended to be a limiting feature of this invention, and the sol may be made by any method delivering a suitable sol. Preferably, the sol will have a weight ratio of aluminium to chloride of at least 13:1.

A preferred method for effecting the gelation of the sol comprises the steps of mixing the sol with a gelling agent at a temperature below the gelation temperature, and then dispersing the resulting mixture as droplets in a hot oil bath, whereby gelation occurs with the formation of firm spherical gel particles. The alumina hydrogel spheres are then subjected to certain ageing treatments in order to impart the desired physical characteristics. Generally, a complete ageing treatment comprises ageing in hot oil for a period of at least 10 hours, ageing in a suitable liquid alkaline medium for at least 10 hours, and finally washing with water to reduce the concentration of alkaline medium. In such a process for the forming and ageing of alumina particles, the hydrogel spheres are not to be contacted with water before being aged in the liquid alkaline medium. The spheres are water-soluble at these earlier stages of the process, and can be destroyed upon contact with water. The ageing treatment may be effected at a temperature of from 49 to 260°C (120 to 500°F). Above 100°C (212°F) there is a tendency for the rapid evolution of gases which could cause the hydrogel spheres to rupture and otherwise become weak. By maintaining a superatmospheric pressure during the forming and ageing step, higher temperatures may be employed for ageing. The utilization of higher temperatures offer such advantages as the elimination of ageing in a liquid alkaline solution. The spheres may therefore be washed with water immediately following the oil-ageing step. Typically, gelled particles are aged in an oil bath for from 1 to 24 hours at a temperature from 90 to 150°C (194 to 302°F) and at a pressure from atmospheric to 150 psig (1034 k Pag). If oil-aged under atmospheric pressure conditions, the gelled particles are generally further aged in a dilute aqueous ammoniacal solution for 2 to 4 hours. After being aged, the particles are water-washed, dried, and calcined.

The gelation of the alumina hydrosol may be effected by mixing the sol with hexamethylenetetramine (HMT), a weak base having a strong buffering action at a pH from 4 to 10. This material also has an increased rate of hydrolysis at increased temperature without a sudden evolution of gas, which is advantageous in the gelation procedure. It is also known that a mixture of urea and HMT may be employed as the gelling agent. Upon heating this mixture to an elevated temperature, the gelling agent decomposes and forms ammonia, which causes the hydrosol to set to a gel and permits the formation of alumina hydrogel spheres. Following gelation and ageing, the particles may be oven-dried at 110°C (230°F) and then heated gradually to 650°C (1202°F), and calcined in air at this temperature for 2 hours. The resultant material, after the air calcination, is essentially gamma alumina. The term "essentially" means that the resultant alumina support comprises at least 90 wt. % of gamma alumina. To ensure that the support material is essentially gamma alumina, it is highly desirable that the support material should not be exposed to a temperature above 850°C (1562°F). Exposure to temperatures above 850°C (1562°F) will result in a phase change of the alumina, converting it from gamma- alumina into delta-, theta-, and possibly even alpha-alumina. Such a phase change is usually accompanied by a collapse of the small pores (less than 100 Angstroms) creating larger pores which results in an increase in total pore volume, but, because the surface area is directly proportional to the quantity and pore size of the small pores, the collapse of these pores results in a dramatic drop in the surface area of the support material. Therefore, by utilizing the oil drop method, it is possible to form a gamma alumina support material having a total pore volume greater than 1.4 cm³/g with a surface area in excess of 150 m²/g, thus avoiding the attendant problems just described associated with alternative forming techniques.

Although not completely understood, utilization of the oil drop method for preparation of the alumina support material yields a superior support as compared to other forming techniques known in the art, for example, extrusion. It has been found that when the oil drop method is employed, it is possible to produce alumina supports having the required total pore volume of greater than 1.4 cm³/g and still maintain a high surface area of greater than 150 m²/g. Other forming techniques are inadequate in that in order to achieve increased total pore volume, the supports must be heat treated, for example, by calcination. Such treatment causes a steep reduction in the surface area of the support, however. Typically, these non-oil-dropped supports cannot achieve total pore volumes of more than 1.0 cm³/g.

Further teaching on the formation of the preferred alumina spheres by the oil drop method may be obtained by reference to US-A-3096295, - 3926849 and - 4250058. The formation of spheroidal alumina particles is also addressed in US-A-4179408, - 4179411 and 4514511.

Besides the basic alumina support material, there are two other components which are important to the performance of the catalyst of this invention. Firstly, there is a nickel component, which may be present only on the outer surface of the alumina support material or uniformly throughout the support. Having the nickel on the outer surface of the support means that the nickel is surface-deposited, such that substantially all of the nickel is concentrated within the outermost 200 micron layer of the support. The concentration of nickel in the finished catalyst is preferably from 1 to 25 wt. %, most preferably from 5 to 15 wt. %, on the basis of the elemental metal. The nickel component can be added to the catalyst during the sphere-formation procedure if it is so desired. It is greatly preferred, however, that the nickel component of the catalyst is added to the previously formed alumina spheres by impregnation, in which the formed alumina spheres are immersed in a solution of a nickel compound. Preferably, the formed calcined alumina spheres are immersed in an aqueous solution of nickel nitrate, nickel chloride, nickel sulphate or nickel acetate, or other water-soluble nickel compound. The solution is then preferably evaporated to dryness in contact with the spheres utilizing a rotary steam evaporator. The dried particles may then be calcined at a temperature of about 150°C (302°F) for 1 hour and then at about 525°C (977°F) for 1 hour. The formed spheres may then be dried and purged with nitrogen and are preferably subjected to a reduction step in contact with a hydrogen-containing gas.

Also preferably present in significant amounts only upon the surface of the alumina support is the sulphur component which is preferably present in an amount from 0.05 to 1.5 wt. %. A preferred range of the sulphur concentration is from 0.1 to 1.0 wt. %, with it being highly preferred that the sulphur concentration in the finished catalyst is less than 0.5 wt. %. The sulphur component is preferably added to the catalyst composite in a final preparation step, after the formation of an alumina base and the placement of the nickel upon the alumina base material. In this sulphiding procedure, the initial composite is subjected to sulphiding conditions sufficient to provide the desired sulphur concentration. Sulphiding may be performed under liquid phase conditions, but it is greatly preferred to perform this step under vapour phase conditions. It is therefore preferred to effect the deposition of the sulphur component upon the catalyst by contacting the initial composite with a vaporous sulphur-containing compound. Preferably, the sulphur-containing compound is present in admixture with hydrogen. A preferred sulphur-containing or sulphur-yielding compound is hydrogen sulphide. Other sulphur or sulphide-yielding compounds which may be employed for this purpose include ammonium sulphide, ammonium hydrosulphide, the alkyl and aryl mercaptans, organic thioethers, disulphides, thioaldehydes, and other sulphur-yielding organic compounds. The sulphiding conditions will therefore preferably include a temperature sufficient to maintain the sulphiding compound present as a vapour, with these temperatures ranging from 10 to 500°C (50 to 932°F). A preferred range is from 20 to 400°C (68 to 752°F) when hydrogen sulphide is utilized as the sulphiding agent. The pressure employed during the sulphiding step can be selected from an extremely broad range and does not greatly affect the course of the sulphiding step. Atmospheric pressure is preferred. The initial composite may be sulphided statically using hydrogen-hydrogen sulphide, or in a flowing gas stream containing 5 to 30% of hydrogen sulphide, which is passed over the initial catalyst composite at a gas hourly space velocity of 2.5 to 10.

The preferred embodiment of the catalyst of the invention is also specific as to those compounds which preferably are not present within the catalyst composite. The catalyst may therefore be characterized herein as being "essentially free" of certain elements. As used herein, the term "essentially free" is intended to indicate a lack of any intent specifically to include the specified element or group of elements in the catalyst. It is recognized, however, that due to the impurities present in industrial grade chemical supplies, and also due to the contamination possible during manufacturing steps in catalyst manufacturing plants which are employed to produce a variety of different catalyst compositions, some contamination of the catalyst with undesired materials will unavoidably occur. The term "essentially free" is therefore employed herein not to indicate a total lack of the specified element or group of elements, but to indicate that the concentration of the specified element or group of elements is less than 0.1 wt. % of the total finished catalyst.

Preferably, the catalyst is essentially free of the noble or platinum group metals, which include platinum, palladium, rhodium, and iridium. Preferably, the catalyst is essentially free of all Group VIII metals except for nickel. Therefore, the catalyst is essentially free of iron and cobalt. Furthermore, the catalyst composite is preferably essentially free of the alkaline earth metals, of which calcium, strontium, and barium are the most common. The catalyst composite should also be essentially free of the alkali metals, of which lithium, sodium, and potassium are the most commonly used catalyst components. Finally, it is also preferred that the catalyst composite is essentially free of the halogens including fluorine, chlorine, bromine, and iodine.

A preferred embodiment of the catalyst composite may therefore be characterized as a catalyst for the selective hydrogenation of diolefinic hydrocarbons which is essentially free of halogens and noble metals, and which comprises an alumina support material having a total surface area greater than 150 m²/g, with less than 25% of the total pore volume of the catalyst being provided by pores having average pore diameters of less than 300 Angstroms and with over 60% of the pore volume being provided by pores having average pore diameters greater than 600 Angstroms, and also comprises 1 to 25 wt. % of nickel and 0.1 to 1 wt. % of sulphur.

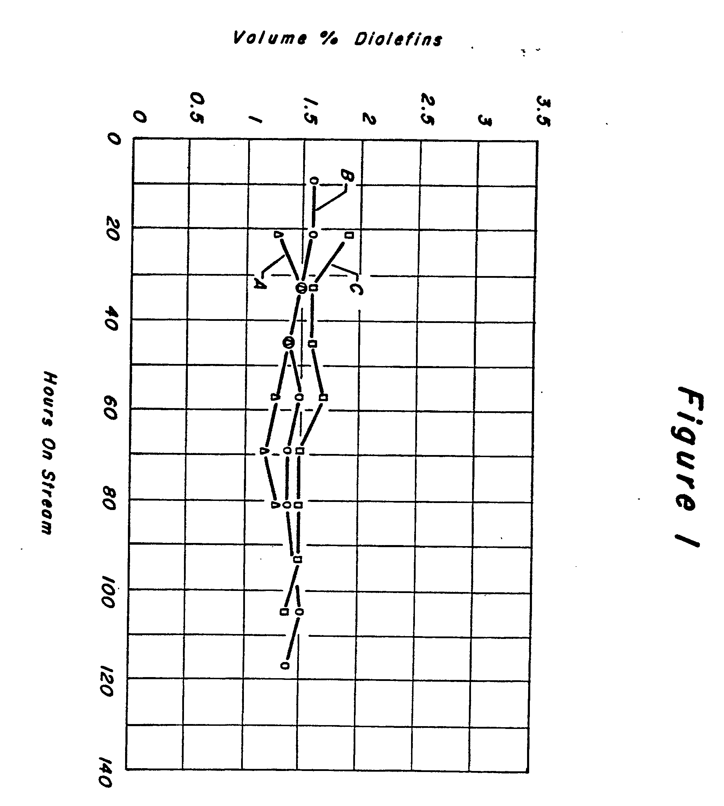

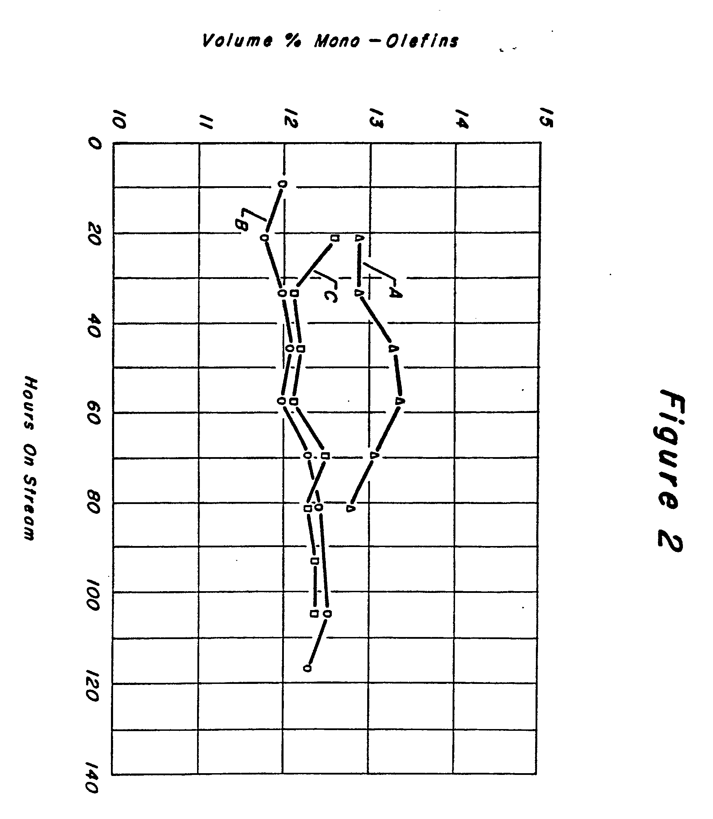

The superior performance of the catalyst of this invention is demonstrated by the data provided in Figures 1 and 2. The data in these figures were arrived at by testing three different catalysts under identical conditions in the same pilot plant, to determine their effectiveness for the selective hydrogenation of diolefinic hydrocarbons in a feed stream comprising a mixture of paraffinic and olefinic hydrocarbons. The feed stream is believed representative of that which would be produced by a commercial dehydrogenation process consuming a feed material comprising $C_{10}$ to $C_{13}$ hydrocarbons. The feed stream contained about 0.99 vol. % of aromatics, with the aliphatic portion of the feed stream comprising 0.1 vol. % of $C_9$ -, 11.2 vol. % of $C_{10}$ -, 26.5 vol. % of $C_{11}$ -, 34.5 vol. % of $C_{12}$ -, 27.6 vol. % of $C_{13}$ -, and 0.1 vol. % of $C_{14}$ - compounds. The feed stream contained a total of 85.46% of saturates, 11.46% of monoolefins, and 2.09% of diolefins. The remainder of the feed stream consisted of non-normal hydrocarbons. The feed stream was first stored in a feed tank under a pressurized atmosphere of high purity hydrogen until the feed liquid was saturated with hydrogen. In trials, the feed stream was then contacted with the respective catalysts at a liquid hourly space velocity of 5 m.⁻¹, a temperature of 200° C (392° F), and a pressure of 100 psig (689 k Pa g). The effluent of the pilot plant was periodically sampled and analyzed to provide the information plotted on Figures 1 and 2. It should be noted that the time on-stream plotted along the X-axis or abscissa of the Figures is actually time measured after the catalyst had been on-stream for a total of 552 hours.

The compositions of the three catalysts tested in the procedure just described are given in the following Table. Catalyst A corresponds to the preferred catalyst composition of this invention. Catalyst B is an alumina-based catalyst comprising a support material also formed by the preferred oil drop method. Catalyst C is based on an extruded base materials comprising a mixture of 85 wt. % of alumina and 15 wt. % of clay. As may be seen from Figure 1, the preferred catalyst composition of Catalyst A produces a treated hydrocarbon effluent which has a lower concentration of diolefinic hydrocarbons than Catalysts B and C, which contain similar amounts of nickel and which have been sulphided in a similar manner. It is, however, more important to direct attention to the information provided in Figure 2, which indicates that the catalyst composition of this invention is more effective at producing a higher concentration of the desired monoolefins than the two comparison catalysts. On comparison of the information provided by Figures 1 and 2, it may be observed that Catalyst A is much more selective at effecting the hydrogenation only of diolefinic hydrocarbons as compared to Catalysts B and C. The results are clearly indicative of Catalysts B an C effecting the hydrogenation of monoolefinic hydrocarbons present in the feedstream in addition to hydrogenation of diolefinic hydrocarbons. It is therefore evident that a process employing Catalyst A would result in higher yields of the desired product, whether this product is simply the monoolefinic hydrocarbon or is a product which results from the consumption of the monoolefinic hydrocarbon, such as an alkylation process for the production of linear alkylbenzene.

By referring to the Table, it may be seen that Catalyst A is unique in its pore size distribution. Specifically, it has a much lower percentage of the total pore volume present in micropores (>300 Angstroms) and most of its pore volume present in macropores (<600 Angstroms). It may be observed that most of the macropore volume is in pores less than 1500 Angstroms in diameter. In comparison, the reference catalysts have significant percentages of their pore volume present in micropores. The preferred catalyst also differs in having a total pore volume which is much larger than the reference catalysts.

The finished catalyst of this invention should have a total pore volume greater than 1.4 cm³/g. Preferably, the catalyst will have a pore volume greater than 1.4 cm³/g but less than 3 cm³/g. As previously stated, less than 25%, and preferably less than 20%, of this volume is to be provided by pores having an average pore diameter, as measured by mercury intrusion, of less than 150 Angstroms. Preferably, less than 30% of the total volume is provided by pores having an average diameter less than 300 Angstroms. The preference for a large amount of macropores is shown by the requirement that over 60% is provided by pores having an average diameter greater than 600 Angstroms. The presence of a few exceptionally large pores, which could provide this amount of volume, is not preferred. Rather, the size distribution of the macropores of the catalyst of this invention is preferably rather narrow. Pores having pore diameters from 600 to 1500 Angstroms should account for at least 50% of the total pore volume. More preferably, 60% of the total pore volume is provided by macropores having mean pore diameters from 600 to 1500 Angstroms.

**TABLE**

| Catalyst | A (Invention) | B (Comparison) | C (Comparison) |
|---|---|---|---|
| Base Composition | Alumina | Alumina | Alumina + clay |
| Ni, wt. % | 9.5 | 9.9 | 9.6 |
| S, wt. % | 0.27 | 0.10 | 0.22 |
| Surface Area, $m^2/g$ | 174 | 195 | 262 |
| Total Pore Volume, $cm^3/g$ | 1.63 | 0.732 | 0.882 |
| **% Pore Volume in Pores** | | | |
| > 1500 Angstroms | 1.47 | 0.34 | 20.0 |
| > 1000 Angstroms | 46.2 | 0.34 | 22.1 |
| > 600 Angstroms | 65.9 | 1.16 | 24.5 |
| > 500 Angstroms | 67.3 | 1.38 | 25.4 |
| > 300 Angstroms | 79.0 | 2.9 | 29.9 |
| > 150 Angstroms | 85.5 | 26.0 | 43.6 |

**Claims**

1. A hydrogenation catalyst which comprises an alumina support material, from 0.05 to 1.5 wt. % of sulphur and 1.0 to 25 wt. % of nickel, characterized in that the alumina support material has a total pore volume from 1.4 to 3 $cm^3/g$, and a surface area greater than 150 $m^2/g$, with less than 25 percent of the total pore volume being provided by pores having pore diameters of less than 150 Angstroms and with over 60 percent of the pore volume being provided by pores having pore diameters greater than 600 Angstroms.

2. A catalyst according to Claim 1 characterized in that less than 30 percent of the total pore volume is provided by pores having an average pore diameter of less than 300 Angstroms.

3. A catalyst according to Claim 1 or 2 characterized in that at least 50 percent of the total pore volume is provided by macropores having mean pore diameters from 600 to 1500 Angstroms.

4. A catalyst according to any one of Claims 1 to 3 characterized in that the alumina support material is prepared by the gelation of an alumina sol.

5. A catalyst according to Claim 4 characterized in that the gelation of the alumina sol is performed in accordance with the oil drop method.

6. A catalyst according to any one of Claims 1 to 5 characterized in that the alumina support material is substantially gamma alumina.

EP 0 307 520 B1

7. A method of selectively hydrogenating a diolefinic hydrocarbon to a monoolefinic hydrocarbon by contacting the diolefinic hydrocarbon and hydrogen under hydrogenation conditions with a catalyst, characterized in that the catalyst is a catalyst according to any one of Claims 1 to 6.

8. A method according to Claim 7 characterized in that the hydrogenation conditions include a pressure of 276 to 5516 kPag, a temperature of 25 to 350°C and liquid hourly space velocity of 1 to 35 hr.⁻¹.

9. A method according to Claim 7 or 8 characterized in that the ratio of hydrogen to diolefinic hydrocarbon is 1:1 to 1.8:1.

**Revendications**

1. Catalyseur d'hydrogénation, qui comprend un support en alumine, 0,05 à 1,5% en poids de soufre et 1,0 à 25% en poids de nickel, caractérisé en ce que le matériau support en alumine a un volume total de pores de 1,4 à 3 cm³/g et une aire spécifique supérieure à 150 m²/g, avec moins de 25% du volume total des pores consistant en pores de diamètres inférieurs à 150 Å et plus de 60% du volume des pores consistant en pores de diamètres supérieurs à 600 Å.

2. Catalyseur selon la revendication 1, caractérisé en ce que moins de 30% du volume total des pores est fourni par des pores ayant un diamètre moyen de moins de 300 Å.

3. Catalyseur selon l'une des revendications 1 ou 2, caractérisé en ce qu'au moins 50% du volume total des pores est fourni par des macropores ayant un diamètre moyen de 600 à 1500 Å.

4. Catalyseur selon l'une quelconque des revendications 1 à 3, caractérisé en ce que le matériau support en alumine est préparé par gélification d'un sol d'alumine.

5. Catalyseur selon la revendication 4, caractérisé en ce que la gélification du sol d'alumine est effectuée selon le procédé de la goutte d'huile.

6. Catalyseur selon l'une quelconque des revendications 1 à 5, caractérisé en ce que le matériau support en alumine est essentiellement de l'alumine gamma.

7. Procédé d'hydrogénation sélective d'un hydrocarbure dioléfinique en hydrocarbure monooléfinique par mise en contact de l'hydrocarbure dioléfinique et d'hydrogène avec un catalyseur dans des conditions d'hydrogénation, caractérisé en ce que le catalyseur est un catalyseur selon l'une des revendications 1 à 6.

8. Procédé selon la revendication 7, caractérisé en ce que les conditions d'hydrogénation sont une pression de 276 à 5516 kPa, une température de 25 à 350°C et une vitesse spatiale horaire du liquide de 1 à 35 h⁻¹.

9. Procédé selon l'une des revendications 7 ou 8, caractérisé en ce que le rapport hydrogène/hydrocarbure dioléfinique vaut 1:1 à 1,8:1.

**Ansprüche**

1. Ein Hydrierkatalysator, bestehend aus einem Aluminiumoxidträgermaterial, 0,05 bis 1,5 Gew.-% Schwefel und 1,0 bis 25 Gew.-% Nickel, dadurch gekennzeichnet, daß das Aluminiumoxidträgermaterial ein Porenvolumen von insgesamt 1,4 bis 3 cm³/g besitzt und eine Oberfläche, die größer ist als 150 m²/g, wobei weniger als 25 Prozent des gesamten Porenvolumens von Poren gebildet werden, deren Porendurchmesser weniger als 150 Angström beträgt, und mehr als 60 Prozent des Porenvolumens von Poren gebildet werden, deren Porendurchmesser mehr als 600 Angström beträgt.

2. Ein Katalysator nach Anspruch 1, dadurch gekennzeichnet, daß weniger als 30 Prozent des gesamten Porenvolumens von Poren gebildet werden, deren Porendurchmesser im Durchschnitt weniger als 300

8

Angström beträgt.

3.  Ein Katalysator nach Anspruch 1 oder 2, dadurch gekennzeichnet, daß wenigstens 50 Prozent des gesamten Porenvolumens von Makroporen gebildet werden, deren Porendurchmesser im Durchschnitt 600 bis 1500 Angström beträgt.

4.  Ein Katalysator nach einem der Ansprüche 1 bis 3, dadurch gekennzeichnet, daß das Aluminiumoxidträgermaterial durch Gelieren eines Aluminiumoxidsols hergestellt wird.

5.  Ein Katalysator nach Anspruch 4, dadurch gekennzeichnet, daß das Gelieren des Aluminiumoxidsols nach der Öltropfenmethode durchgeführt wird.

6.  Ein Katalysator nach einem der Ansprüche 1 bis 5, dadurch gekennzeichnet, daß das Aluminiumoxidträgermaterial im wesentlichen Gamma-Aluminiumoxid ist.

7.  Ein Verfahren zur selektiven Hydrierung eines diolefinen Kohlenwasserstoffs in einen monoolefinen Kohlenwasserstoff, indem man den diolefinen Kohlenwasserstoff und Wasserstoff unter Hydrierungsbedingungen mit einem Katalysator in Kontakt bringt, dadurch gekennzeichnet, daß der Katalysator ein Katalysator nach einem der Ansprüche 1 bis 6 ist.

8.  Ein Verfahren nach Anspruch 7, dadurch gekennzeichnet, daß die Hydrierungsbedingungen einen Druck von 276 bis 5516 kPag, eine Temperatur von 25 bis 350°C und eine Raumgeschwindigkeit der Flüssigkeit von 1 bis 35 pro Stunde umfassen.

9.  Ein Verfahren nach Anspruch 7 oder 8, dadurch gekennzeichnet, daß das Verhältnis von Wasserstoff zu diolefinem Kohlenwasserstoff 1 : 1 bis 1,8 : 1 beträgt.

Figure 1

Figure 2

Volume % Mono — Olefins

Hours On Stream